# EUROPEAN PATENT APPLICATION

(11) **EP 2 392 327 A1**
(43) Date of publication of application: **07.12.2011**
(21) Application number: 10075240.1
(22) Date of filing: 04.06.2010
(51) Int. Cl.: A61K 31/365, A61P 33/00

(54) **Use of orlistat for the anti-parasitic treatment of a parasitic disease**

(71) Applicant: Universitätsmedizin der Johannes Gutenberg-Universität Mainz, 55131 Mainz (DE); Max-Planck-Gesellschaft zur Förderung der Wissenschaften e.V., 80539 München (DE)
(72) Inventor: Petry, Franz, Prof. Dr., 65343 Eltville (DE); Hedberg, Christian, Dr., 44227 Dortmund (DE)
(74) Representative: Arth, Hans-Lothar

(57) **Abstract**

The present invention relates to the field of medicine, parasitology and microbiology and more particularly to the use of Orlistat as a therapeutic agent for preventing, treating or eliminating a protozoal and parasitic disease in an animal including a human.

## Description

### Field of the invention

The present invention relates to the field of medicine, parasitology and microbiology and more particularly to the use of Orlistat as a therapeutic agent for preventing, treating or eliminating a parasitic disease in an animal including a human.

### Background of the invention

The empirical formula of the compound Orlistat is C₂₉H₅₃NO₅, and its molecular weight is 495.7 Da. It is an enantiopure single diastereomeric molecule that contains four stereogenic centers with a negative optical rotation in ethanol at 529 nm. Orlistat, also known as tetrahydrolipstatin, was originally derived semi-synthetically from lipstatin, which is isolated from the bacterium Streptomyces toxytricini. It is a white to off-white crystalline powder with a waxy, fluffy and sticky appearance, and having a low melting point about 44°C. Orlistat is practically insoluble in water, freely soluble in chloroform, and very soluble in methanol and ethanol. Orlistat has no pKa within the physiological pH range, due to the lack of organic acid or base functionalities in the molecule. The chemical structure of Orlistat is as follows:

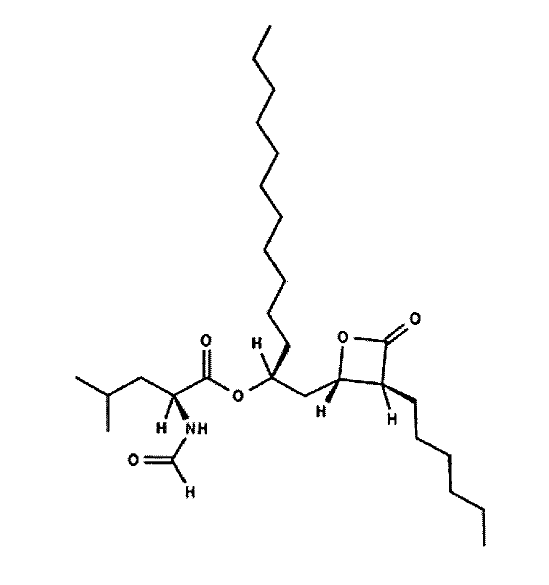

Orlistat has been originally disclosed in the US-Patent US4598089 and in the European patent EP129748 in the year 1984. In these and other patents and patent applications various pharmaceutical formulations are disclosed.

Orlistat is a reversible covalent lipase inhibitor, acting as a slow turnover substrate. It exerts its therapeutic activity in the lumen of the stomach and small intestine by forming a covalent bond to the active site nucleophilic serine residue of gastric and pancreatic lipase. The inactivated enzymes are thus unavailable to hydrolyze dietary fat in the form of triglycerides into absorbable free fatty acids and monoglycerides. Undigested fats are not absorbed, and the resulting caloric deficit may have a positive effect on weight control.

Orlistat has been used mainly for the treatment of adiposity and obesity by reducing the fat intake. No publications concerning the treatment of parasitic infections and diseases were found.

Objective of the present invention is to provide a pharmaceutically active agent useful to treat parasitic infections.

This objective is solved by providing Orlistat as an anti-parasitic agent. Further preferred embodiments of the present invention are disclosed in the dependent claims, the description and the examples.

Surprisingly it was found that Orlistat is highly active against a protozoon and infections caused by a protozoon

### Description of the invention

Parasitism is a type of symbiotic relationship between organisms of different species where one organism, the parasite, lives in or on a host organism and gets its food from or at the expense of the host. In general, parasites are much smaller than their hosts, show a high degree of specialization for their mode of life, and reproduce more quickly and in greater numbers than their hosts. Classic examples of parasitism include interactions between vertebrate hosts and diverse animals such as single celled protozoa, multicellular worms (helminthes) and arthropoda. Parasites reduce the fitness of the host by a general pathology, food competition, organ damage, modification of the behavior, modifying the appearance thereby influencing human social life, and others. Often parasite infections of low intensity parasite persist without doing harm and causing symptoms. Medium and heavy infections however can be the cause of severe disease manifestations, thereby inflicting morbidity and mortality.

Surprisingly it was found that Orlistat is effective as anti-infective agent against a protozoan parasite. Consequently one aspect of the present invention is directed to the use of Orlistat for the manufacture of a pharmaceutical composition for prophylaxis and/or treatment of a parasitic infection. Orlistat is also useful for prophylaxis and/or treatment of a disease caused by a parasitic infection. Such diseases which can be treated with Orlistat are infections which are caused by a parasitic organism or parasite

Parasites include, but are not limited to *Acanthamoeba, Balantidium, Blastocystis, Coccidia, Cryptosporidium, Cyclospora, Dientamoeba, Eimeria, Entamoeba, Enterocytozoon, Enzephalitozoon, Giardia, Hammondia, Isospora, Leishmania and Microsporidia.*

Parasitic infections cause a tremendous burden of disease in both the tropics and subtropics as well as in more temperate climates. Parasitic infections which are common in developed countries in temperate climates include *Trichomonas, Giardia, Cryptosporidium* and *Toxoplasma.*

As used herein the term infection refers to the invasion, colonization and multiplication of a foreign species in body tissue, especially that causing local cellular injury due to competitive metabolism, toxins, intracellular replication, or antigen-antibody response. The host usually responds to an infection by inflammation.

As used herein the term disease refers to an impairment of the normal state of a living animal or any of its components that interrupts or modifies the performance or the function of the animal or its components and is a response to specific infective agent such as parasites. Typical parasitic diseases include, but are not limited to, amebiasis, balantidiasis, blastocystis infection, coccidiosis, cryptosporidiosis, dientamebiasis, microsporidiasis, giardiasis, leishmaniasis and isosporiasis.

Cryptosporidiosis is a diarrheal disease caused by microscopic parasites of the genus *Cryptosporidium,* which are protozoa of the phylum Apicomplexa. Both the disease and the parasite are commonly known as "Crypto." It affects the intestines of mammals and is typically an acute short-term infection. Many species of *Cryptosporidium* exist that infect humans and a wide range of animals. The parasite is protected by an outer shell that allows it to survive outside the body for long periods of time and makes it very resistant to chlorine disinfection. While this parasite can be transmitted in several different ways, water is a common method of transmission and *Cryptosporidium* is one of the most common causes of waterborne disease (drinking water and recreational water) among humans worldwide. Cryptosporidiosis is especially life-threatening in immuno-compromised individuals.

Giardiasis in humans is caused by the infection of the small intestine by a single-celled organism called *Giardia lamblia.* Giardiasis occurs worldwide with a prevalence of 20-30% in developing countries. Additionally, *Giardia* has a wide range of human and other mammalian hosts, thus making it very difficult to eliminate. *Giardia* infects over 2.5 million people annually.

Amoebiasis or amebiasis refers to infection caused by the ameba *Entamoeba histolytica.* A gastrointestinal infection that may or may not be symptomatic and can remain latent in an infected person for several years, amebiasis is estimated to cause 70,000 deaths per year worldwide. Symptoms can range from mild diarrhea to dysentery with blood and mucus in the stool. Severe amebiasis infections (known as invasive or fulminant amebiasis) occur in two major forms. Invasion of the intestinal lining causes amebic dysentery or amebic colitis. If the parasite reaches the bloodstream it can spread through the body, most frequently ending up in the liver where it causes amebic liver abscesses.

The microsporidia constitute a phylum of spore-forming unicellular parasites. They were once thought to be Protists but are now known to be Fungi. Loosely 1500 of the probably more than one million species are named now. Microsporidia are restricted to animal hosts, and all major groups of animals host microsporidia. Most infect insects, but they are also responsible for common diseases of crustaceans and fish. The distinguished species of microsporidia usually infect one specific host or a related group of hosts. Several species, most of which are opportunistic, also infect humans. Microsporidia produce highly resistant spores to survive outside the host for up to several years. In the gut of the host the spore germinates. It builds up osmotic pressure until its rigid wall ruptures at its thinnest point at the apex. The posterior vacuole swells, forcing the polar filament to rapidly eject the infectious content into the cytoplasm of the potential host. Simultaneously the material of the filament is rearranged to form a tube which functions as a hypodermic needle and penetrates the gut epithelium. The microsporidia often cause chronic, debilitating diseases rather than lethal infections.

*Cyclospora cayetanensis* is a protozoan that causes disease in humans, and perhaps other primates. *Cyclospora cayentanensis* is an apicomplexan, cyst-forming coccidian protozoan that causes a self-limiting diarrhea.

Antiparasitics are a class of medications which are indicated for the treatment of parasitic diseases such as nematodes, cestodes, trematodes, infectious protozoa, and amebas. Anti-parasitic are drugs which cause death of a parasitic organism, inhibit its growth or reproduction, paralyze it, prevent attachment to tissue and cells, or inhibit its cell entry.

There are many anti-parasitic drugs available for the treatment of a variety of protozoal and parasitic infections. Common antiprotozoal drugs include, but are not limited to, tinidazole, metronidazole, melarsoprol, eflornithine, rifampin, amphotericin B, pentamidine, sodium stibogluconate, meglumine antimoniate, fluconazole, artesunate, quinine, quinidine, chloroquine, atovaquone-proguanil, artemether-lumefantrine, mefloquine, doxycycline, clindamycin, paromomycin, atovaquone, nitazoxanide, azithromycin, fumagillin, paromomycin, diloxanide, secnidazole, ornidazole, iodoquinol, diloxanide furoate, clindamycin, atovaquone, azithromycin, diminazen, trypan blue, praziquantel, oxamniquinine, niclosamide, albendazole, mebendazole, thiabendazole, pyrantel, diethylcarbamazine, ivermectin, selamectin, doramectin, abamectin.

For the treatment of Cryptosporidium infection, drugs such as atovaquone, azithromycin, paromomycin and nitazoxanide are used. Many parasites developed resistance against various drugs, so that new antiparasitic drugs are needed with mechanisms of action which are different from the known drugs where resistances are known of.

The present invention relates also to a method of treating and preventing parasite infections in a patient comprising administering to said patient an effective amount of Orlistat sufficient to treat said infection. Also diseases caused by said parasite infections can be treated by the administration of Orlistat. The patients which suffer from an infection of a parasite which can be treated with Orlistat are animals, mammals and humans.

Orlistat is useful to treat such an infection of a parasite which means that Orlistat is able to eliminate the parasite from the patient or to destroy or kill the parasite in the patient. Moreover Orlistat is also useful to treat diseases caused by an infection of a parasite.

Furthermore, the present invention describes the use of the drug Orlistat for manufacturing a pharmaceutical composition for prophylaxis and/or treatment of a parasitic infection. According to the invention a pharmaceutical composition containing Orlistat shall be used for the prevention and/or treatment of infections by parasitic agents. Further, according to the invention, the pharmaceutical composition containing Orlisitat shall be used for the treatment of diseases caused by parasite infections, such as, but not limited to *Acanthamoeba, Balantidium, Blastocystis, Coccidia, Cryptosporidium, Cyclospora, Dientamoeba, Eimeria, Entamoeba, Enterocytozoon, Enzephalitozoon, Giardia, Isospora and Microsporidia.* Preferred are the use of the inventive pharmaceutical composition for the prevention, treatment and/or elimination of infections of protozoal parasites, more preferred those of *Cryptosporidium, Cyclospora, Eimeria,* and most preferred those of *Cryptosporidium.*

According to the invention the pharmaceutical composition containing Orlistat shall be used for the prevention and/or treatment of diseases which comprise, but are not limited to, amebiasis, balantidiasis, blastocystis infection, coccidiosis, cryptosporidiosis, dientamebiasis, microsporidiasis, giardiasis, isosporiasis. Preferred are the use of the inventive pharmaceutical composition for the prevention and/or treatment of diseases caused by protozoal agents, more preferred those of cryptosporidiosis, cyclosporiasis, eimeriasis and most preferred those of cryptosporidiosis.

The present invention further describes a method for preventing, treating and/or eliminating a protozoal or parasitic infection in an animal or a human. In accordance with the method, a pharmaceutical composition comprising Orlistat is administered to an animal infected with parasitic agent in an amount to prevent, reduce or eliminate the infection. The pharmaceutical composition can be administered to animals such as, but not limited to, humans and other mammals, birds, fish, reptiles, amphibians and crustaceans to treat or prevent parasitic infection, however, the administration to mammals and humans is preferred.

Orlistat or the pharmaceutical composition containing Orlistat together with substantially nontoxic pharmaceutically acceptable carrier, excipients, adjuvants or diluents can be administered through common routes of administration. The medications of the present invention are prepared in a conventional solid or liquid carrier or diluents and a conventional pharmaceutically acceptable adjuvant at suitable dosage level in a known way. The preferred compositions and formulations are in administratable form which is suitable for oral application. These administratable forms, for example, include pills, tablets, film tablets, coated tablets, capsules, powders, granules, solutions, suspensions in an aqueous or a non-aqueous liquid, oil-in-water liquid emulsion or water-in-oil emulsion and deposits. Other than oral administratable forms are also possible. Orlistat or pharmaceutical compositions or formulations containing Orlistat may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

For the manufacture of pharmaceutical compositions for the use according to the invention Orlistat can be processed with pharmaceutically inert, inorganic or organic carriers. Examples of which can be used for tablets, coated tablets, dragées and hard gelatin capsules are lactose, corn starch or derivatives thereof, talc, stearic acid or its salts and the like. Suitable for soft gelatin capsules are, for example, vegetable oils, waxes, fats, semi-solid and liquid polyols and the like, depending on the nature of the active ingredient no carriers are, however, generally required in the case of soft gelatin capsules. Suitable carriers for the manufacture of solutions and syrups are, for example, water, polyols, saccharose, invert sugar, glucose and the like. Moreover, the pharmaceutical compositions can contain preserving agents, solubilizers, stabilizing agents, wetting agents, emulsifying agents, sweetening agents, coloring agents, flavoring agents, for varying the osmotic pressure, buffers, coating agents or antioxidants. They can also still contain other therapeutically valuable substances and drugs.

It has been surprisingly found that Orlistat inhibits growth or kills or prevents entry of parasites into host cells or host tissue. The effect of the drug Orlistat on *Cryptosporidium* was particularly effective.

The protozoal or parasitic infection treatment method according to the invention is effective for preventing, treating, killing, destroying and/or eliminating parasites and for preventing and/or treating diseases caused by a protozoal and parasitic agent such as, but not limited to *Balantidium, Blastocystis, Coccidia, Cryptosporidium, Cyclospora, Dientamoeba, Eimeria, Entamoeba, Enterocytozoon, Enzephalitozoon, Giardia, Hammondia, Isospora, Leishmania, Microsporidia,* Preferred are the prevention, treatment and elimination of infections of protozoal parasites, more preferred those of *Cryptosporidium, Entamoeba, Giardia, Cyclospora, Microsporidia, Enterocytozoon, Encephalitozoon,* and most preferred those of *Cryptosporidium.*

Diseases which can be prevented and treated by the use and method according to the invention consist of amebiasis, balantidiasis, blastocystis infection, coccidiosis, cryptosporidiosis, dientamebiasis, microsporidiasis, giardiasis, isosporiasis and leishmaniasis. Preferred are the prevention and treatment of diseases caused by protozoal agents, more preferred those of cryptosporidiosis, amebiasis, giardiasis, cyclosporiasis, microsporidiasis, and most preferred those of cryptosporidiosis.

### Description of the figures

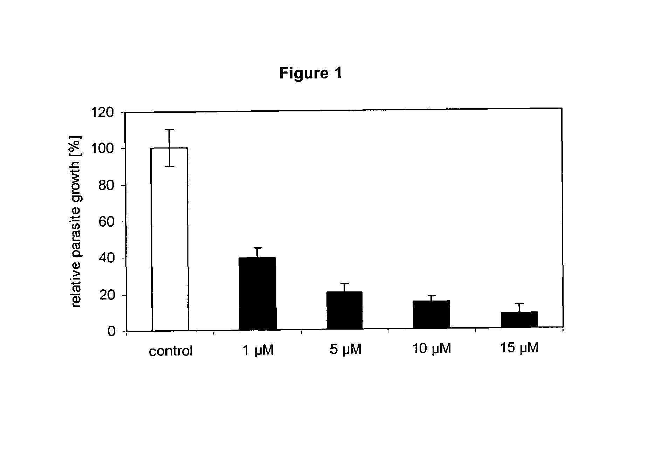

Figure 1: *In vitro* inhibition of the development of *Cryptosporidium parvum* in HCT-8 cells by Orlistat.
   In a cell culture-based enzyme-immune assay increasing concentrations of Orlistat (1 - 15 µM) diluted in culture medium were tested for the inhibition of the development of *Cryptosporidium parvum.* The parasites were allowed to develop in HCT-8 cells for 48 hrs. The optical readings were converted into parasite growth relative to mean of the controls [%]. Error bars represent standard deviations.

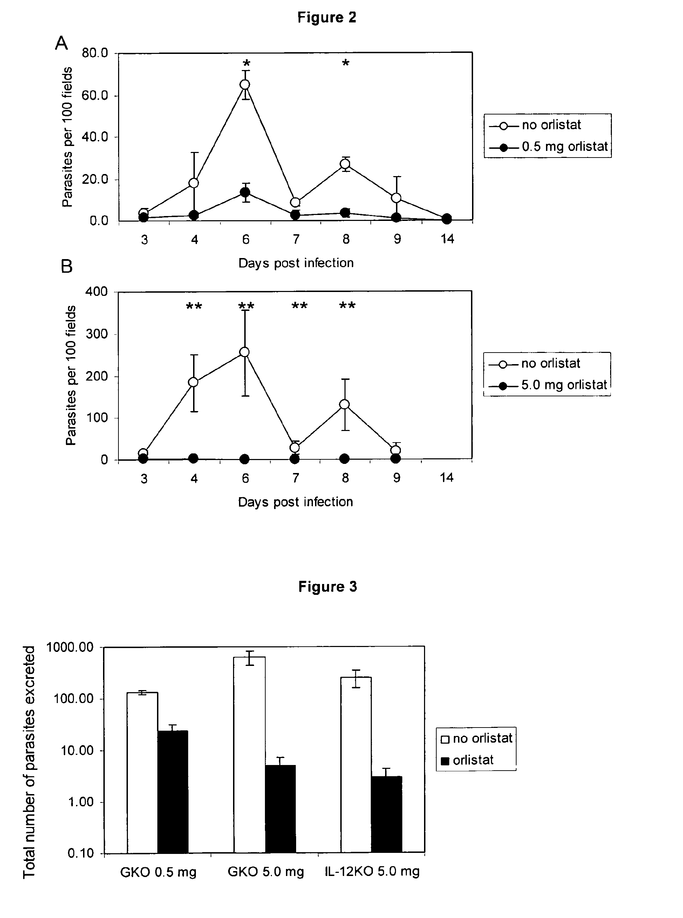

Figure 2: Fecal parasite excretion of *Cryptosporidium parvum*-infected interferon-gamma deficient mice.
   Mice were offered chow paste with 0.5 mg (A) or 5.0 mg (B) Orlistat per gram dry matter. Control mice were offered chow paste without Orlistat. Parasites were detected in fecal smears by immunofluorescent antibody test. 100 microscopic fields (x1000 magnification) were screened. Error bars represent standard deviations.
   Asterisks denote statistical significance between treated and non-treated groups at days indicated (* p < 0.05; ** p < 0.01).
Figure 3: Fecal parasite excretion of *Cryptosporidium* parvum-infected interferon-gamma (GKO) and interleukin-12 (IL-12KO) deficient mice.
   Mice were offered chow paste with 0.5 mg or 5.0 mg Orlistat per gram dry matter. Control mice were offered chow paste without Orlistat. The mean of the total number of parasites excreted over the patent period (day 3 to 14) is shown. The dotted line depicts the practical detection limit (1 parasite per 100 microscopic fields). Error bars represent standard deviations.

### Examples

### Example 1: Pharmaceutical compositions of Orlistat

### Composition A: gelatin capsule

Orlistat, microcristalline cellulose and sodium starch glycolate were blended in a mixer. A solution of polyvinylpyrolidone and sodium lauryl sulfate in water was prepared. This solution was added to the mixture for granulation. The granules are formed into pellets and dried at 30°C. After talcum was added and homogenously distributed these pellets were filled into hard gelatin capsules (each containing 120 mg Orlistat). Table 1 shows the final amount of the ingredients per capsule.

**Table 1: Composition of a gelatin capsule**

| **Compound** | **Amount / Capsule** |
|---|---|
| Orlistat | 120 mg |
| lactose | 80 mg |
| cellulose | 120 mg |
| sodium lauryl sulfate | 11.4 mg |
| sodium starch glycolate | 40 mg |
| polyvinylpyrrolidone (povidon) | 20 mg |
| talcum | 0.4 mg |
| total | 391.8 mg |

### Composition B: chewable tablet composition - fatty acid and Orlistat

Orlistat and myristic acid were melted together at 50°C. Mannitol and lactose were added and the mixture was cooled down to RT (room temperature) under continuously stirring. Talcum was added. The powder was pressed into tablets (each containing 60 mg Orlistat).

**Table 2: Composition of a chewable tablet**

| **Compound** | **Amount / Tablet** |
|---|---|
| Orlistat | 60 mg |
| myristic acid | 30 mg |
| mannitol | 400 mg |
| lactose | 400 mg |
| talcum | 10 mg |
| total | 900 mg |

### Composition C: tablet with a fatty acid salt and Orlistat

The ingredients are mixed with stepwise addition of a (50:50% m/m) ethanol/water mixture (0.2 ml/g). The formed granules are dried in vacuum at 30°C and pressed into tablets (each containing 120 mg Orlistat).

**Table 3: Composition of a tablet with a fatty acid salt and Orlistat**

| **Compound** | **Amount / Tablet** |
|---|---|
| Orlistat | 120 mq |
| sodium laurate | 30 mg |
| mannitol | 80 mg |
| hydroxypropylmethylcellulose | 60 mg |
| total | 290 mg |

### Composition D: tablet with a sucrose fatty acid ester and Orlistat

Orlistat, sucrosepalmitate and maltodextrin were mixed and solved in water. This solution was dried in a way that a powder achieved. Cellactose and talcum were added and homogenously distributed. This mixture was pressed into tablets (each containing 120 mg Orlistat). Table 4 shows the final amount of the ingredients per tablet.

**Table 4: Composition of a tablet with a sucrose fatty acid ester and Orlistat**

| **Compound** | **Amount / Tablet** |
|---|---|
| Orlistat | 120 mg |
| sucrosepalmitate | 120 mg |
| maltodextrin | 750 mg |
| cellactose | 375 mg |
| talcum | 15 mg |
| total | 1380 mg |

### Composition E: Orlistat and bile acid sequestrant

Orlistat was melted and maltodextrin was added. The mixture was cooled down to RT (room temperature) under continuously stirring. Then cholestyramine was added. A solution of aspartame in water was prepared. This solution was added to the mixture for granulation. The granules were dried at 60°C. After blending these granules in a mixer they were filled into sachets (each containing 120 mg Orlistat). Table 5 shows the final amount of the ingredients.

**Table 5: Composition of Orlistat and a bile acid as a sequestrant**

| **Compound** | **Amount** |
|---|---|
| Orlistat | 120 mg |
| maltodextrin | 740 mg |
| cholestyramine | 4000 mg |
| aspatame | 440 mg |
| total | 5300 mg |

### Composition F: Orlistat cream

**Table 6: Orlistat cream**

| **Compound** | **Amount (weight %)** |
|---|---|
| Orlistat | 1 |
| cera | 30 |
| glycerol | 15 |
| polypropylenglycol | 10 |
| sodium acetate | 5 |
| purified water | ad 100 |

### Composition G: Gel with Orlistat

All ingredients were mixed and stirred for 10 min at 60°C. The mixture was cooled down to RT (room temperature). During this process at around 45°C a gel was formed.

**Table 7: Composition of Orlistat in a gel**

| **Compound** | **Amount (weight %)** |
|---|---|
| Orlistat | 5 |
| sodium hydroxide | 6 |
| propandiol-1,2 | 15 |
| glycerol | 3 |
| polyacrylic acid | 10 |
| purified water | ad 100 |

### Example 2: Inhibition of the development of Cryptosporidium parvum by Orlistat in an in vitro system

Orlistat (# 04139, Sigma, Deisenhofen, Germany) was dissolved in DMSO at 10 mM (stock solution).

The ileocecal adenocarcinoma cell line HCT-8 was purchased from the ATCC (CCL244). Cells were passaged in maintenance medium at 37°C in a 6% CO₂/94% humidified air incubator.

HCT-8 cells which served as host cells for the intracellular development of *Cryptosporidium parvum* were seeded in plastic 96-well culture plates (NUNCLON Surface, Nunc, Wiesbaden, Germany). Cytotoxicity of Orlistat was tested in an MTS/PMS assay (Hommer et al., 2003). Orlistat stock solution and DMSO solvent (control) were diluted in culture medium and serial dilutions were applied to HCT-8 cultures for 48 hrs. A cytotoxic effect of Orlistat on HCT-8 cells was detected at concentrations > 20 µM. DMSO had no cytotoxic effect at appropriate dilutions.

HCT-8 cells were seeded in 96-well culture plates and infected with *Cryptosporidium parvum* oocysts (IOWA strain) as described (Hommer et al., 2003). At 2 hrs post infection (p.i.) non-invaded parasites were removed by washing. Orlistat diluted in culture medium or medium alone (negative control) were added. After a total of 48 hrs p.i. culture plates were washed with PBS and fixed with methanol for 15 min. Intracellular stages of *Cryptosporidium parvum* were detected by an enzyme-immune assay using a monoclonal antibody (McDonald et al., 1995) as described previously (Hommer et al., 2003).

Experiments were performed in six-fold replica. Optical density (OD) readings were corrected by the OD of non-infected HCT-8 cells. The means of the infected controls without Orlistat were taken as 100% growth rate. All individual readings from infected and compound-treated HCT-8 cells at each concentration were transformed into relative growth rate [%]. A Student's T-test was performed to test statistical significance between the study groups.

In figure 1 the results of this *in vitro* testing are shown. The treatment with Orlistat resulted in a concentration dependent, statistically significant reduction of parasite development in the HCT-8 host cells for all Orlistat concentration (p < 0.0002) and reached an inhibition rate of > 90% at 15 µM compared to the infected but untreated controls.

### Example 3: Inhibition of the development of Cryptosporidium parvum by orlistat in an in vivo system

As a source of Orlistat, Xenical^{®} capsules (120 mg Orlistat, Roche Pharma AG, Grenzach-Wyhlen, Germany) were used.
Standard extruded chow pellets (SSNIFF, Soest, Germany) were ground into fine powder and mixed with the content of Xenical^{®} capsules. A paste was made of chow powder with or without Orlistat and sterile water (1:1 w/v) and offered to mice *ad libitum.* Mice aged 8-12 weeks with genetic defects of interferon-gamma or interleukin-12 were infected with *Cryptosporidium parvum* oocysts by oral gavage. These strains of mice are susceptible to *Cryptosporidium parvum* infection as adults (Jakobi & Petry, 2008). Mice were housed under specific pathogen free (SPF) conditions. Fecal droppings of each individual mouse were processed for parasite detection using an immunofluorescent antibody test (Jakobi & Petry, 2008).

In the first two rounds of experimental infection interferon-gamma deficient mice (8 mice per study group) were infected with 1x10⁶ parasites and offered chow paste supplemented with 0.5 mg Orlistat per gram dry matter starting the day of infection until the end of the experiment (figures 2A and 3). Mice treated with Orlistat excreted 5 to 7-fold fewer parasites compared to control mice over the whole study period (14 days). The mice accepted the formulated feed and showed no adverse reactions (normal mobility, agility, fur appearance, body weight).

In a third experimental infection using interferon-gamma and interleukin-12 deficient mice the parasite inoculum was raised to 1.5x10⁶ parasites. This resulted in an overall higher infection rate in control mice compared to an inoculum of 1x10⁶ parasites. The Orlistat concentration was increased to 5.0 mg Orlistat per gram dry matter (figures 2B and 3). Increasing the amount of Orlistat resulted in a reduction of total parasite excretion by a factor of 125 in interferon-gamma deficient mice (p < 0.0006) and by a factor of over 80 in interleukin-12 deficient mice (p < 0.008). The number of fecal parasites in Orlistat-treated mice ranged between 0 and 2 parasites per 100 microscopic fields (detection limit: 1 parasite per 100 fields).

## Claims

1. Use of Orlistat or a pharmacologically acceptable salt thereof for the manufacture of a pharmaceutical composition for prophylaxis and/or treatment of a parasitic infection.

2. Use according to claim 1 for prophylaxis and/or treatment of a disease caused by a parasitic infection.

3. Use according to claim 1 or 2, wherein the infection is caused by a parasitic organism.

4. Use according to any one of claims 1 - 3, wherein the infection is caused by a parasitic organism selected from the group comprising *Acanthamoeba, Balantidium, Blastocystis, Coccidia, Cryptosporidium, Cyclospora, Dientamoeba, Eimeria, Entamoeba, Enterocytozoon, Enzephalitozoon, Giardia, Hammondia, Isospora, Leishmania and Microsporidia.*

5. Use according to any one of claims 1 - 4, wherein the infection is caused by a single-celled parasitic organism.

6. Use according to any one of claims 1 - 5, wherein the infection is caused by a parasitic organism selected from the group comprising *Cryptosporidium, Eimeria, Entamoeba, Giardia, Cyclospora, Microsporidia, Enterocytozoon and Encephalitozoon.*

7. Use according to claim 6, wherein the infection is caused by *Cryptosporidium.*

8. Use according to any one of claims 1 - 7 for eliminating a protozoal or a parasitic agent from a patient having a protozoal or a parasitic disease.

9. Use according to claim 8 wherein the patient is a vertebrate or a crustacean.

10. Use according to claim 8 wherein the patient is a mammal.

11. Use according to claim 10 wherein the mammal is a human.

12. Use according to any one of claims 1 - 11, wherein the disease is selected from amebiasis, balantidiasis, blastocystis infection, coccidiosis, cryptosporidiosis, cyclosporiasis, dientamebiasis, microsporidiasis, giardiasis, leishmaniasis and isosporiasis.

13. Use according to claim 12 wherein the disease is caused by an infection by cryptosporidiosis, amebiasis, giardiasis, cyclosporiasis or microsporidiasis.

14. Use according to claim 13 wherein the disease is cryptosporidiosis.

15. A method of treating and preventing parasite and protozoa infections in a patient comprising administering to said patient an effective amount of the Orlistat sufficient to treat said infection.
